Europäisches Patentamt

European Patent Office

Office européen des brevets

(19)

(11) Publication number: **0 152 254**
**A2**

(12) **EUROPEAN PATENT APPLICATION**

(21) Application number: **85300736.7**

(22) Date of filing: **04.02.85**

(51) Int. Cl.⁴: **G 01 N 33/53**
**G 01 N 33/542**

(30) Priority: **06.02.84 US 577552**

(43) Date of publication of application:
**21.08.85 Bulletin 85/34**

(84) Designated Contracting States:
**BE DE FR GB IT LU NL**

(71) Applicant: **E.I. DU PONT DE NEMOURS AND COMPANY**
**1007 Market Street**
**Wilmington Delaware 19898(US)**

(72) Inventor: **Lambert, Stephen Burton**
**P.O. Box 723**
**Groton Massachusetts 01450(US)**

(74) Representative: **Myerscough, Philip Boyd et al,**
**J.A.Kemp & Co. 14, South Square Gray's Inn**
**London, WC1R 5EU(GB)**

(54) Chromogenic support immunoassay utilizing labeled complement components.

(57) An immunoassay, utilizing an active support capable of interacting with a substantially colorless product, formed in a reaction between an enzyme-labeled productive complex and enzyme substrates, for form a signal-emitting product associated with the support, is provided. The productive complex is formed between an immune complex of an analyte with its enzyme-labeled antibody and an enzyme-labeled complement component. The immune complex is either formed or collected on the active support during the assay. The assay is useful for the detection and measurement of analytes in biological samples.

EP 0 152 254 A2

Croydon Printing Company Ltd.

## Title

CHROMOGENIC SUPPORT IMMUNOASSAY UTILIZING
LABELED COMPLEMENT COMPONENTS

### TECHNICAL FIELD

This invention relates to immunoassay techniques for the detection and quantification of bacteria, viruses, and soluble antigens and, more specifically, to immunoassay techniques in which a labeled immune complex is collected on an active support and in which the signal produced by the label component is concentrated on the support material by virtue of its chemical or physical interaction with the support.

### BACKGROUND ART

Clinical laboratory chemical diagnostic tests are an important component of health care delivery. In recent years, a large number of immunoassay techniques have been described which take advantage of the specificity of antigen-antibody reactions for the measurement of soluble organic antigenic or haptenic compounds, including drugs, hormones, vitamins, metabolites, etc., in body fluids.

Numerous label components for producing a detectable signal in immunoassays have been described in the patent and nonpatent literature, including radioisotopes, enzymes, fluorophores, enzyme inhibitors, chemi- or bio-luminescent materials, etc. See, for example, Methods in Enzymology, Volumes 70 and 73 (1980 and 1981), H. van Vunakis and J. Langone, ed., New York, Academic Press and references contained therein.

Some immunoassays require a separation step in order to distinguish the bound from the free label component. Such assays are said to be heterogeneous and include such well-known techniques as the radioimmunoassay (RIA) and the enzyme-linked immunosorbent assay (ELISA). A variety of means of separation is known in the art, such as centrifugation, filtration, and chromatography.

Other immunoassays do not require a separation step and are said to be homogeneous. Homogeneous assays, while faster and simpler to perform, are frequently less sensitive than heterogeneous assays and can be more prone to interferences.

In conventional immunoassays of either the homogeneous or heterogeneous type, the reaction product(s) of the label components is free to diffuse into the test medium. This facilitates spectrophotometric detection since the products can be uniformly distributed throughout the test volume. However, dilution of the reaction product(s) limits sensitivity and makes direct visualization difficult.

While immunoassays have been widely used for the measurement of soluble antigens and haptens, traditional diagnostic methods for infectious diseases have involved the cultivation of the infectious agent _in vitro_ or in laboratory animals. This approach suffers from several disadvantages. First, some medically important viruses cannot be cultured in commonly available tissue culture or animal systems. These include rotavirus, hepatitis A and B viruses, and Epstein-Barr virus. Second, many infectious agents require long periods of cultivation before identification can be accomplished. In some cases this period may be so long that the results are not useful to the clinician. Third, traditional cultivation methods can be inadequate when specimens are obtained from individuals who have already received antibiotic therapy.

There is a rapidly expanding market for assays capable of detecting infectious agents directly in clinical samples. Such assays can be based on antigen-antibody reactions or on measurement of microbial enzyme activity. It should be noted that assays based on antigen or enzyme detection do not necessarily measure live or infectious organisms. For any such assay to be practically useful, it is essential that the assay have a rapid turnaround time, be specific for the organism or class of organisms of interest, be sensitive to a relatively small number of organisms, and be relatively free of interferences from components encountered in body fluids.

A number of immunoassays for microbial and viral antigens have been described; see R. H. Yolken, Rev. Infect. Dis., Volume 4,35 (1982), for a review. Despite their advantages over traditional methods of cultivation, many of these assays are still comparatively time-consuming (1-3 hours) and labor intensive (requiring multiple reagent additions and washing steps). Because the generated signal is measured in solution, inadequate sensitivity is also often a problem.

U.S. Patent 4,327,073, issued April 27, 1982 to Huang, discloses a method for the quantitative analysis of biological fluids for a plurality of substances, each of which undergoes at least one reaction with a respective cognate compound. The substrate carrier which is used for immobilizing the functional reactive compound (antibody or nonantibody binding protein) can have a functional group for covalent bonding of the reactive compound or ion exchange sites for ionic bonding of the functional reactant. The substrate carrier can also be an adsorbent in which case the reactive compound is attracted by Van der Waals forces. Enzymes are used as the tracers and are measured by bathing the carrier in a chromogenic substrate for the enzyme; the resultant product is a precipitated colored solid.

U.S. Patent 4,322,495, issued March 30, 1980 to Kato, discloses an immunoassay for antibodies to a microorganism which utilizes a nonporous ceramic or polymer support on which the organism of interest is immobilized. The support is incubated with a sample suspected of containing antibody to the organism, washed by flooding to remove unbound components, and incubated with an enzyme-labeled anti-antibody. After another wash, an indicator capable of reacting with the enzyme label is applied to produce a color. The colored product does not interact physically or chemically with the support.

U.S. Patent 3,187,075, issued February 5, 1980 to Noller, describes an immunoassay for hepatitis B virus which utilizes an antibody-coated, inert, solid support to capture virus from the sample. After washing, the bound virus is reacted with a second, enzyme-labeled antibody and a substrate for the enzyme label. The colored product of the reaction precipitates on the nylon support and is eluted with a mixture of saline and acetone for measurement.

Canadian Patent 1,104,927, issued July 14, 1981 to Cole et al., discloses a method for the detection of a component of an immunochemical reaction in which one component is immobilized on a microporous membrane. It is reacted with sample containing the second component of an immunochemical reaction, followed by an enzyme-labeled immunochemical reactant for the second component. The amount of color change on the surface of the membrane is determined following reaction of the enzyme label with a chromogen. The chromogen can precipitate on the membrane when acted on by the enzyme label or it can bind preferentially to the membrane. Precipitation on or preferential binding of the chromogen to the support is largely fortuitous.

U.S. Patent 4,200,690, issued April 29, 1980 to Root et al., discloses an immunoassay for an intestinal parasite in which the test result is measured by the color which forms on a support. The chromogenic substance used is poorly soluble in the reaction solvent or is preferentially entrapped in the filter matrix in the colored state. The precipitation on or preferential binding of the chromogen to the support is largely fortuitous.

The use of the Clq component of the complement cascade adsorbed to the wells of a polyvinyl microtiter plate for capture of immune complexes was described by Yolken et al., Journal of Clinical Microbiology, Volume 11,546 (1980). The Clq is utilized to capture immune complexes of cytomegalovirus and goat anticytomegalovirus. An enzyme-labeled anti-antibody was used in a heterogeneous immunoassay to detect the presence of immune complex. This approach has the disadvantages that it lacks sufficient specificity and sensitivity due to the general nature of unlabeled Clq binding to immune complexes that are not derived from the analyte of interest; the authors ranked the Clq-based assay tied for fifth place among seven immunoassays.

A Clq-enzyme conjugate was used in an assay for soluble immune complexes, W. H. Stimson et al., J. Clin. Lab. Immunol., Volume 5,129 (1981). Heat-aggregated, insolubilized IgG was used to capture Clq-HRP conjugate which was bound to soluble IgG immune complexes in decomplemented human serum. The chromophore produced by enzyme-substrate interaction is in solution in dilute form thereby limiting assay sensitivity.

U.S. Patent 4,174,384, issued November 13, 1979 to Ullman, et al., describes a homogeneous variation of a simultaneous sandwich immunoassay. In that assay, both antibodies are employed in soluble form and both are labeled, one with a fluorescer and the other with a quencher. When both antibodies are bound to antigen, fluoroscence quenching occurs. Fluorescence-based immunoassays have the disadvantages that they require sophisticated instrumentation for detection of the signal and are subject to interferences from fluorescent materials present in commonly assayed biological fluids.

U.S. Patent 4,299,916, issued November 10, 1981 to Litman, et al. discloses the use of complementary enzyme pairs in close proximity, including glucose oxidase and horseradish peroxidase, which intereact to produce an insoluble chromophore preciptated on a surface. The color intensity is related to selected analytes in solution. One of the enzymes is bound to one member of an immunological pair, while the second enzyme is added in solution along with signal generating reagent or is initially bound to the solid phase prior to its exposure to sample containing analyte. In this homogeneous immunoassay, the immunochemically bound reagents are not separated from those which remain free in solution in order to complete the assay by detection of a signal. This assay suffers from reduced specificity in that only one member of the complementary enzyme pair is usually bonded to an immunoreactive species. The second enzyme is added passively in solution or on a solid phase, increasing the potential for non-specific reactivity in a homogeneous procedure. This procedure also compromises the sensitivity of the immunoassay by raising non-specific signal levels from soluble species in the surrounding medium. The sensitivity of the homogeneous immunoassay is further limited by the binding constants of the immunoreactive species because the assay is generally conducted in a competitive mode. The precipitation of the chromophore generated onto the passive solid phase is largely fortuitous and there is no suggestion of signal amplification on the solid phase.

U.S. Patent 4,233,402, issued November 11, 1980 to Maggio et al describes a homogeneous enzyme channeling immunoassay. In that assay, human IgG (huIgG) is labeled with glucose oxidase (GO) and anti-huIgG is labeled with horseradish peroxidase (HRP). One of the products of the GO-catalyzed breakdown of glucose, $H_2O_2$, serves as a substrate for HRP and hence the amount of signal produced is a function of the proximity of the labeled components. Addition of increasing amounts of unlabeled huIgG as sample results in decreasing signal. The possibility of performing a sandwich assay in which two antibodies to the compound of interest are labeled, each with one member of an enzyme channeling pair, is also disclosed. This assay suffers from the disadvantage that the signal producing means is free to diffuse into the test medium. While this facilitates spectrophotometric detection, the resultant dilution of the signal limits the sensitivity of the assay and makes direct visualization of the assay result difficult.

PCT/US83/01887 (EP-A-126772) discloses the use of an active support to concentrate the signal generated by a label component in a heterogeneous immunoassay. Concentration of the signal on the support makes possible its visualization. If the signal were uniformly distributed in solution, it would be substantially indistinguishable over background because of its dilution in the assay medium.

There is a need for an improved immunoassay method for antigens, especially of viral or bacterial origin, which is rapid, sensitive to the small numbers of organisms present in biological samples in the early stages of infections, and is potentially quantitative.

## DISCLOSURE OF THE INVENTION

The chromogenic support immunoassay of this invention for the detection or measurement of analytes such as soluble organic antigens and haptens, cells, microorganisms, and their products in biological samples utilizes a system in which the analyte is ultimately contacted with an enzyme-labeled antibody. The immune complex formed by analyte and enzyme-labeled antibody is contacted in turn with at least one serum complement component which is labeled with an enzyme different from that used to label the antibody. Both enzymes utilized for labeling are selected for their complementary interreactivity in which the product of one enzyme's catalytic reaction with substrate serves as the substrate for the second enzyme. The product of this second enzyme reaction, in turn, interacts with an active support to form a signal-emitting product.

The generation of signal by this assay system is dependent upon the presence and appropriate spatial positioning of both enzyme-labeled components. If only one enzyme-labeled component were present during treatment with signal-generating reagents because appropriate specific binding events have not occured, then no signal would be generated. Useful signal can be produced only if there is binding (fixation) of at least one enzyme-labeled complement component to the immune complex. Such a configuration will be defined as a productive complex to differentiate it from an immune complex, the latter defined here as the b'nding pair comprising analyte and enzyme-labeled antibo y.

The chromogenic support immunoassay of this invention comprises the following steps:

(A) collecting or forming on an active support an immune complex having a first enzyme component comprising an analyte immunochemically bound to an antibody and a productive complex having a second enzyme component comprising at least one complement component which is bound to the immune complex;

(B) washing the active support to remove unreacted enzyme-labeled binding species;

(C) contacting the enzyme label components on the support with signal-generating reagents whereby a substantially colorless product is formed that is soluble in the assay medium; and

(D) allowing the product to interact with the support which interaction results in the formation of a signal-emitting product associated with the support.

The labeled immune complex can be formed either in solution or directly on the active solid support. When formed in solution by pre-incubation of the analyte with an enzyme-labeled antibody it is then reacted to form a productive complex with an enzyme-labeled complement component, which binds only to antibody participating in a reaction with its specific analyte, and which is subsequently collected on a support. Direct formation of the productive complex on the support can involve immunochemical capture of an immune complex, formed by preincubation of analyte and enzyme-labeled antibody, by an enzyme-labeled complement component bound to the support. Alternatively, the productive complex can be formed by having either analyte or enzyme-labeled antibody bound to the active support sequentially exposed to the complementary member of the immune complex binding pair and then to at least one enzyme-labeled complement component.

The products formed by the complementary action of the two labeling enzymes closely positioned in the productive complex of this invention are soluble and are substantially colorless until they interact with the active support. This interaction with said support can be chemical, physical, or ionic to form a signal-emitting product.

Another aspect of this invention is further signal enhancement by the use of additional enzyme-labeled complement components which bind in sequence to the immune complex and to each other. The specific, sequential binding events that occur in the serum complement system are used to attach multiple enzyme labels to the immune complex, thereby increasing the substrate turnover capabilities of the productive complex and the sensitivity of the chromogenic support immunoassay.

## DESCRIPTION OF THE INVENTION

The chromogenic support immunoassay of this invention is applicable to the detection and measurement of a variety of analytes such as soluble organic antigens and haptens, cells, microorganisms, and their products. Soluble organic antigens and haptens include synthetic antigenic peptides, drugs, hormones, vitamins, food additives and pesticides. The microorganisms include bacteria, mycoplasma, rickettsia, chlamydia, and viruses. The products can be synthetic such as enzymes, toxins, colicins, or cell surface antigens or breakdown products such as antigenic fragments of viral coat proteins. Some bacteria of interest are staphylococci, streptococci, neisseria, and the Enterobacteriaciae. Some viruses of interest are herpes simplex virus, Epstein-Barr virus, cytomegalovirus, myxoviruses, adenoviruses, and hepatitis viruses. When the analyte is an antigen or enzyme produced by a cell or microorganism, it can be found on the surface of living or dead cells or organisms, in extracts of cells or organisms, or as cellular debris. The presence of analyte in patient sample, therefore, does not necessarily correlate with infectivity.

The assay of this invention utilizes an active support designed to fulfill the functions of concentrating the chromatic signal generated by the label components. In addition, the support can also serve as a carrier for signal generating reagents and for the analyte either by physical entrapment on or within the support matrix or by immunochemical capture mediated by support attachment of one member of a binding pair of the immune or productive complexes.

By active support is meant a support capable of binding to or associating with the reaction components of the immunoassay of this invention and with the products of the reactions between the enzyme labels and the signal generating reagents.

Concentration of the signal by the support makes possible its visualization which, were it uniformly distributed in solution, would be substantially indistinguishable over background because of the high dilution of the signal in the assay medium. Direct visual readout of a qualitative test result is thus possible. The results can also be read quantitatively for example by densitometry or semi-quantitatively by visual comparison to a series of standards of varying color intensities.

In addition to the intensity of color concentrated on the support, the localization of color can also be informative. For example, when a patient sample suspected of containing the analyte of interest is pre-incubated with the antibody-enzyme conjugate and then applied to a support, the localization of signal on the support is detected by addition of a second enzyme-labeled reagent comprising at least one component of the complement cascade. This concentrated signal corresponds to the localization of analyte. This phenomenon can be exploited for comparative purposes to relate the number of live or infectious organisms, determined by traditional cultivation methods, to the presence of antigenic material contained in the sample.

The support can be fabricated from a variety of porous materials. Among suitable supports are included polyamides such as nylon; starch granules; starch-coated materials such as cotton or filter paper; cellulose nitrate; polyvinyl acetate and partially hydrolyzed polyvinyl acetate, and poly-(acrylamide)/starch; other synthetic or natural polymers; modified polyamides; and mixtures thereof. Nylon mesh discs can be hydrolyzed with, for example, HCl, to yield functional groups, for example, $-NH_2$ and $-COOH$, for possible further reactions. Nylon can be further treated with a crosslinking reagent such as XAMA-7, a trifunctional aziridine compound from Cordova Chemical Co., or hexanediamine.

The support can be fabricated from nonporous materials also such as ceramic beads, dipsticks, test tubes and granular materials where porosity of the granules would not be required for carrying out the immunoassay. The support can also contain other materials capable of binding to or associating with antigens, their enzyme-labeled antibodies, or enzyme-labeled serum complement components. It is sometimes advantageous to coat the above materials with latex polymers to which proteins can be attached covalently or noncovalently. It is further possible to prepare the active support by mixing the porous materials with other carriers capable of binding to or associating with antigens, their enzyme-labeled antibodies, or enzyme-labeled complement components. These carriers include glass beads, Sephadex cross-linked dextrans, Sepharose cross-linked agaroses and Zorbax® packing materials (a registered trademark of E. I. du Pont de Nemours & Company).

The support itself can have any appropriate physical form on which to perform an assay. The form is dictated by the circumstances of the assay and can be disc-like such as a filter disc, elongated such as a swab or a dipstick, columnar, rectangular, etc. In general, a porous or microporous support is preferred since the kinetics and efficiency of capture of the reactive components and of washing are maximized. While macroporous supports can be suitable in some circumstances, microporous supports with average pore size <1μm diameter are generally preferred because of the increased surface area available for reaction. Pore size can also be dictated by the dimensions of the various immune complexes formed during the assay. For general use, it is preferred that the pore size of the support be less than 10 nm or greater than 10 μm. The choice of the smaller pore size would eliminate any intraparticle entanglement of viral antigens present in biological samples (most viruses are approximately 18-250 nm in size). The choice of the larger pore size would not lead to size-related fractionation of even bacteria (average size of the generally rod-shaped organisms is 0.5 µm x 2 µm) by the particles. As a general requirement, the porosity of the support material should be such as to avoid fractionation of the antibody-enzyme conjugates (approximate molecular weight $10^4$-$10^6$ daltons).

The assay of this invention can be performed on a disc-shaped porous support, in a column packed with an active support, or on a swab made of a suitable support material. Each of the above can be adapted to test for the presence of a single analyte or can be subdivided into multiple areas, each adapted to test for the presence of a different analyte when specific binding reagents are bound to the support for analyte capture. Where the assay is performed on a swab, the swab on which the assay is performed can be the same one used in obtaining patient sample. Where the assay is performed on a disc or in a column, patient sample is customarily collected in a separate vessel and an aliquot withdrawn for assay.

Nonspecific binding to the support can be reduced or eliminated by pretreating the support with a dilute protein solution (e.g., albumin) which saturates the nonspecific attachment sites or by including in the various reagent and wash solutions a low concentration of a nonionic surfactant.

The interaction of the support with the product(s) generated by the reaction of the enzyme components with their substrates must occur rapidly and selectively. The concentration of the chromatic signal on the support can occur through the intervention of physical forces or chemical bonding. The bonding can be ionic or covalent.

Physical forces such as hydrogen bonding, van der Waals forces, and hydrophobic interactions can participate in the attachment of the reaction products between the enzyme labels and signal generating reagents to various support materials. Ionic properties of the support can be tailored to complement the ionic properties of the reaction product(s). For example, cationic supports can be employed to affix anionic products, and vice versa. Some examples of acidic polymers are polystyrene sulfonic acid, carboxymethyl cellulose, and copolymers of acrylic, methacrylic and maleic acids. Some examples of basic polymers are polypeptide, copolymers of amino-functional monomers, and diethylaminoethyl cellulose.

Covalent attachment of the reaction product(s) to the support can be achieved in different ways. An advantage of this approach is enhanced color permanence, since the products become an integral part of the support.

The support can be prepared to contain functional groups for the covalent attachment of the reaction products. These groups are parts of color couplers which are known in the photographic arts. Examples include azomethine, azo-, leuco-, indazolin-, indoaniline- and pyrazalone dyes.

In order to prepare supports with color couplers, these need to have low mobility. They can be made nondiffusing by increasing their molecular weight through the addition of hydrophobic and/or ionic groups. This results in surfactant-like structures which can be incorporated directly into a polymeric support. The couplers can also be immobilized on the support by covalently bonding the couplers to the support.

In some cases, it is desirable to coat the support with labeled anti-analyte antibodies or complement components in order to specifically capture analyte or immune complexes, respectively, from the sample. In general, it is preferable to attach the antibody or complement components covalently to the support, either directly through a functional group on the antibody or complement component, or indirectly through a spacer arm such as a protein, polyamino acid, or synthetic linking group. Adsorption of the antibody or complement component can sometimes afford sufficient stability during the assay, although covalent attachment is thought to be desirable from the standpoint of stability. The method of attachment should be chosen so as to preserve the binding activity to the highest degree possible. When labeled anti-analyte antibodies are utilized in this manner, the antibody utilized in the enzyme-labeled antibody conjugate can be the same as that used to coat the support or it can be an antibody directed to different antigenic determinants on the analyte.

In some cases, where the analyte is an antibody, the cognate antigen can be attached to the support for subsequent immunochemical capture of the antibody.

In some other cases, where the productive complex is formed in the assay medium, such as by pre-incubation of the sample containing the analyte with an enzyme-labeled antibody followed by enzyme-labeled complement component(s), the support must be of such pore size to retain the productive complex formed but to permit excess reagents to be passed through. Commercially available filter papers or porous membranes allow the convenient selection of a variety of suitable pore sizes. One particularly suitable porous membrane has a nominal pore size of $0.45\mu$.

An important consideration in binding an active species comprising enzyme-labeled antibody or complement component to the active solid support for immunochemical capture, is to conduct the binding in a manner to retain both the specific binding properties of the antibody or complement component and the catalytic properties of the enzymes bound to them. It may be desirable in some circumstances to bind the active species directly to the support or through the use of an appropriate intermediate substance linking the active species to the solid support.

Antibodies needed for the performance of the immunoassay of this invention can be produced by methods well known in the art, including animal immunization and cell fusion (hybridoma) techniques. It can be used as whole serum, ascites or tissue culture fluid, but it is generally preferred to use a purified or partially purified preparation. An immunoglobulin fraction can be prepared by ammonium sulfate precipitation; an IgG fraction can be obtained by ionexchange chromatography or gel filtration. Antibodies can also be affinity purified by elution from an antigen-coated support.

The complement fixing or binding antibodies which can be used in this invention include human $IgG_1$, $IgG_2$, and $IgG_3$, mouse IgG2a and 2b, guinea pig IgG2, rabbit IgG, and IgM antibodies from species known to possess complement fixing antibodies. When IgG-type antibodies are selected to practice this invention, they must have specificity for or be directed against analytes having at least two epitopes, where epitope is understood to mean a single determinant. The IgM-type antibodies selected can be directed against analytes with one or more epitopes.

In the immunoassay of this invention, the labeled nonantibody protein used to capture immune complexes on solid supports or as a pre-incubation reagent to bind immune complexes in solution, or to capture the productive complexes so formed, is at least one member of the serum complement proteins which interact with immunoglobulin molecules that have participated in a specific binding event with analyte or with each other. These complement proteins can be Clq, Clr, and Cls for soluble and insoluble, membrane-bound analytes, each having an enzyme label preferably covalently attached, which does not interfere.with the specific binding of said complement proteins to immune complexes and to each other. It is known that Clr binds to Clq and Cls binds to Clr.

These complement proteins serve a dual function in this invention. They enlarge the molecular dimensions of the immune complexes attached to them, in the form of a productive complex, in order to insure physical entrapment on or within pore size-controlled solid phases. Second, they act as carriers for an appropriate enzyme which participates in reactions with the complementary enzyme label of the antibodies in close proximity.

The complement proteins must be purified from appropriate animal sources, for example, according to the method of Yonemasu et al., J. Immunol., Volume 106,304 (1971), or can be purchased in purified form. When used as pre-incubation reagents in association with labeled antibody, labeled complement proteins are held in media substantially devoid of $Ca^{++}$ and $Mg^{++}$ cations to prevent nonspecific adsorption or activation. Calcium and magnesium ions can be provided in the assay analyte dilution buffer added to mediate the binding of labeled complement protein to immune complexes.

The enzymes in the labeled antibody and complement component conjugates can be chosen from a variety of complementary enzyme pairs known in the art such as those in the following table:

| First Enzyme | Second Enzyme |
|---|---|
| Glucose oxidase | Horseradish peroxidase |
| Galactose oxidase | Horseradish peroxidase |
| Uricase | Horseradish peroxidase |
| Glucose oxidase | Microperoxidase |
| Esterase | ß-glucuronidase |
| Alkaline phosphatase | Peroxidase |
| Hexokinase | Glucose-6-phosphate dehydrogenase |
| Alkaline phosphatase | ß-galactosidase |

The enzymes are chosen for their stability, turnover number, ease of purification, ease of measurement, and freedom from interfering substances often found in biological samples. Moreover, they are especially chosen for their complementarity, in which the product of the first enzyme-substrate reaction serves as the substrate for a second enzyme. The product from this reaction, in turn, interacts with the solid support to generate and concentrate signal in the immunoassay of this invention. When the analyte is an enzyme, it can act both as a label and as a member of a specific binding pair.

The conjugation of the enzyme label to an antibody or complement component can be carried out by known methods and in such a way as to minimize both the loss of immunoreactivity of the antibody or complement component and the loss of activity of the label. Attachment can be direct or indirect, via a spacer arm. Usually, it is necessary to purify the conjugate before use in the assay in order to remove any unreacted label, unlabeled antibody and complement component, and nonimmunoreactive labeled antibody and complement component. This can be accomplished by a combination of gel filtration and affinity chromatography steps.

In general, it is desirable to have as many enzyme molecules per active binding species and in no case should the ratio of enzyme to active binding species be less than 1:1. The number of enzyme label molecules is limited by the overriding need to maintain both immunoreactivity of the active binding species and enzymatic reactivity of the label. In some cases, it can be advantageous to link the active binding species and the label each to a common carrier rather than to each other. For example, one antibody or complement protein and several labels can be conjugated to a high molecular weight protein or polysaccharide, such as dextran.

The reaction product(s) of the enzyme component of the productive complex with signal generating reagents is substantially colorless and is capable of interacting with the support. This interaction leads to the formation of a signal-emitting product associated with the support. The signal-emitting product, if existed independently of the support, would be soluble in the assay medium. The interaction is made possible by the matching of the support material and the substantially colorless product(s) and makes the assay of this invention uniquely useful for the rapid determination of agents causing infectious diseases. The signal generating reagents are those components of the immunoassay of this invention which are necessary to produce a detectable signal upon interaction of a product of the enzyme label components of the productive complex with the active support. These reagents include substrates for the enzyme labels or a combination of materials capable of ultimately producing a substrate for the enzyme labels such as in coupled enzyme reactions.

The product is substantially colorless, that is, not sufficiently visible for a practical assay but can become more visibly colored upon interaction with the support through the formation of a signal-emitting product. For example, the support can be starch or a starch-coated material which can complex with iodine generated as the product of an enzyme-catalyzed reaction to yield a characteristic blue color on the support. Here, the signal-emitting product is a starch-iodine complex.

The assay of this invention can be carried out by first contacting patient sample suspected of containing analyte with a preincubation reagent comprised of an anti-analyte antibody - enzyme conjugate and an enzyme-labeled complement component such as Clq. Patient sample can include whole blood, blood serum, blood plasma, urine, cerebrospinal fluid, a throat swab, lesion exudate, an aqueous dispersion of feces, and discharges, scrapings or lavages.

When patient samples contain native, unlabeled complement components, it is preferred in some instances that such samples be decomplemented to remove interfering complement components that would combine with immune complexes and, in so doing, displace labeled complement components added during the assay. A convenient decomplementation method is described by Stimson et al., J. Clin. Lab. Immunol., Volume 5,129 (1981).

In other instances, it is preferred that samples are not decomplemented. The displacement of specific labeled complement components from immune complexes by native complement components can be utilized in the assay of this invention to provide an evaluation of deficiencies or excesses of said native complement components in patient samples relative to standards of known concentrations.

Preincubation can be typically carried out in a medium buffered at pH 7-8, most often in the range of pH 7.2-7.5, with $Ca^{++}$ and $Mg^{++}$ ion concentrations each in the range of 0.1-1.0 μM (preferably 0.5-1.0 μM for $Ca^{++}$ and 0.1-0.4 μM for $Mg^{++}$), in the temperature range of 4-45°C, most often 23-37°C, over a period of one minute to one hour, most often five to fifteen minutes. The active species resulting from this pre-incubation is referred to as the productive complex. At least a molar excess of both antibody-enzyme conjugate and enzyme-labeled Clq over the analyte is used to insure adequate antigen-antibody reaction and complement fixation in the short time allowed. Reaction stoichiometry can be determined empirically.

Additional enzyme molecules can be incorporated into the productive complexes of soluble analytes by addition of enzyme-labeled Clr and Cls, respectively.

The productive complex can be deposited on the support by filtration, spotting or swabbing. The support can be then washed with an aqueous solution buffered to a pH between 4-10. The buffer can also contain surfactant such as nonionic surfactant, protein(s) such as albumin, and electrolytes. Washing can be accomplished by flowing buffer through the support or, in some cases, it can be sufficient to wash the support by repeated immersions in fresh buffer. The former approach is usually faster and more efficient and is preferred. Free conjugates are not captured by the support while conjugates bound to analyte are retained on the support and are available for subsequent reaction with the signal generating reagent. Extraneous substances in the sample that have not participated in the immunochemical reactions are also removed by washing.

The assay of this invention can also be carried out by covalently attaching the anti-analyte antibody-enzyme conjugate to the active solid support and sequentially contacting this support with patient sample to form an immune complex followed by Clq-enzyme conjugate to form the productive complex. The support can then be washed as described above.

The assay of this invention can also be carried out by covalently attaching the labeled complement component, preferably Clq-enzyme conjugate, to the active solid support and contacting this support with immune complexes formed upon pre-incubation of patient sample with anti-analyte antibody-enzyme conjugate. Conditions for pH, time, and temperature of preincubation and subsequent washing are as above.

After washing, the support is contacted with signal generating reagent. When the antibody-enzyme label is horseradish peroxidase (HRP) and the Clq-enzyme label is glucose oxidase (GO), for example, the signal generating reagent consists of glucose and sodium iodide in a suitably buffered aqueous medium. The signal generating reagent can be flowed through the support or the support can be immersed in the solution. $H_2O_2$ generated by the action of glucose oxidase on glucose serves as a substrate for HRP, generating a hydroperoxide complex. This, in turn, oxidizes iodide ion to iodine which forms a signal-emitting complex with the amylose in starch to form a deep blue color. This color, the intensity of which is directly proportional to the concentration of analyte in the sample, is concentrated on the support. Preferably, the color formed is stable and can be kept as a permanent record of the test result.

Amplification of the signal-emitting product is possible through additional sequential treatment of a soluble productive complex based on labeled Clq with enzyme-labeled Clr and enzyme-labeled Cls reagents which bind to immune complex-bound Clq-enzyme conjugate in the order of the addition. These additional enzyme-labeled complement proteins increase the molecular dimensions of the productive complex and also add to the specific activity of the complex. For each Clq-enzyme conjugate bound to an immune complex, two Clr-enzyme conjugates and two Cls-enzyme conjugates can be bound to it.

## EXAMPLE 1

### ASSAY FOR HERPES SIMPLEX VIRUS

A. Glucose Oxidase Conjugation to Clq

Glucose oxidase (Sigma Chemical Co.; St. Louis, Mo.) was conjugated to Clq (CBR Laboratories, Inc.; Boston, Mass.) in a variation of the two-step glutaraldehyde procedure of Avreamas et al., Immunochem., Volume 8,1175 (1971). Ten milligrams of glucose oxidase was dissolved in 0.2 mL of 0.1M phosphate buffer, pH 6.8, containing 1.25% (v/v) glutaraldehyde. The mixture was held with occasional agitation by gentle rocking motion at room temperature for 18 hours. The mixture was then chromatographed on a 1x60cm column of Sephadex G-50 (Pharmacia Fine Chemicals; Piscataway, N.J.) prepared with 0.15M NaCl. The column was eluted with 0.15M NaCl and the brown colored zone containing the enzyme collected in a 1 to 2 mL volume. One milligram of Clq was added in 1mL 0.15M NaCl and the pH of the resulting solution was adjusted to 9.5 with 0.1mL of 1.0M carbonate/bicarbonate buffer. This solution was held at 4°C for 24 hours when 0.1mL of a 0.2M lysine solution was added to react with any remaining active aldehyde over an additional 24 hour period at 4°C. The glucose oxidase-Clq conjugate prepared in this fashion was dialyzed against several changes of borate-buffered saline (0.1M boric acid; 0.025M sodium tetraborate; 0.075M sodium chloride; pH 7.6) until the pH was reduced to 7.6 from 9.5. The dialyzed conjugate solution was stored at -20°C.

B. Preparation of Active Solid Support

Easy-On Spray Starch was sprayed into a beaker and allowed to settle. Bovine serum albumin (BSA;RIA grade; Sigma Chemical Co., St. Louis, Mo.) was mixed to a final concentration of 1.0% (w/v) with the starch suspension. Nitrocellulose microporous filters (Type HA; 0.45 μM; 47mm, Millipore Corp., Bedford, Ma.) were submerged in the starch-BSA solution, removed, and dried at 40°C. Prepared filters were stored at room temperature in air-tight brown bottles for up to two months prior to use, when they were rehydrated in the borate-buffered saline utilized in Example 1(A).

C. Assay

(1) Clq-Enzyme Conjugate Level Selection

Selected quantities of rabbit anti-HSV-1 conjugated to horseradish peroxidase (HRP-anti-HSV-1; Accurate Scientific; Westbury, N.Y.) and the glucose oxidase (GO)-Clq conjugate prepared as in (A) above were diluted together in veronal buffer diluent (0.14M sodium chloride; 0.0055M sodium-5,5-diethylbarbituate; 0.0035M hydrochloric acid; 0.1% (w/v) gelatin) pH 7.3-7.4, devoid of $Ca^{++}$ or $Mg^{++}$ cations. The HRP-anti-HSV-1 was used at a final dilution of 1:30 in each of several test solutions in which the GO-Clq conjugate final dilution ranged from 1:4 to 1:128. To 50 μL of each of the GO-Clq dilutions containing a constant amount of HRP-anti-HSV-1 was added 50 μL of HSV-1 antigen in tissue culture fluid (M.A. Bioproducts;

Walkersville, Md.) as a 1:50 dilution in veronal buffered diluent containing 0.00056M magnesium chloride and 0.00150M calcium chloride. Fifty microliters of each dilution was also combined with 50 μL of the veronal buffer diluent containing $Mg^{++}$ and $Ca^{++}$ but no HSV-1 as negative reaction controls. Each solution was gently mixed and allowed to incubate at room temperature for 30 minutes. Following incubation, 50 μL of each solution was applied to a starch coated filter as prepared in (B) above and washed with 30mL of a phosphate buffered saline containing Tween 20 surfactant (0.05% v/v). Each filter was immersed in a Petri dish containing 1 mL of a color generating solution (0.04M citric acid; 0.067M disodium phosphate; 0.056M glucose; 0.094M sodium iodide; pH 5.5) at room temperature for 10 minutes. The color generating solution was removed after 10 minutes. Color (blue) estimates were visually graded on a scale of - (negative) to 4 +; "bv" signifies a "barely visible" color which can be just differentiated from a negative reading but less intense than a +/- reading. Visual readings were taken on the filters at 5, 10, and 15 minutes from the time the color generating solution was first in contact with each filter with the following results:

|                    | Color Intensity | | | | | |
|--------------------|-----------------|------------------|------------|------------------|------------|------------------|
|                    | 5 minutes | | 10 minutes | | 15 minutes | |
| Clq-GO Dilution    | Positive | Negative Control | Positive | Negative Control | Positive | Negative Control |
| 1:4                | +2 | – | +3 | – | +4 | bv |
| 1:8                | +2 | – | +3 | – | +4 | bv |
| 1:16               | +1 | – | +2 | – | +3 | bv |
| 1:32               | +/– | – | +/– | – | +1 | – |
| 1:64               | bv | – | bv | – | +/– | – |
| 1:128              | – | – | bv | – | bv | – |
| no Clq-GO          | – | – | – | – | – | – |

A Clq-GO level which maximizes signal over background was chosen between 1:4 and 1:8 final dilution.

(2)  Anti-HSV-1-HRP Level Selection

All conditions, procedures, reagents, and concentrations used in (C)(1) above were also used here except that the anti-HSV-1-HRP conjugate was diluted over a range of 1:10 to 1:60 with Clq-GO used at a constant 1:5 dilution.  Readings were taken at 5 and 10 minutes following addition of the color generating solution with these results:

| Anti-HSV-1-HRP Dilution | Color Intensity | | | |
| | 5 minutes | | 10 minutes | |
| | Positive | Negative Control | Positive | Negative Control |
| --- | --- | --- | --- | --- |
| 1:10 | 4+ | +/- | 4+ | +/- |
| 1:20 | 3+ | +/- | 4+ | +/- |
| 1:30 | 2+ | - | 3+ | +/- |
| 1:40 | 2+ | - | 3+ | +/- |
| 1:50 | 1+ | - | 2+ | +/- |
| 1:60 | 1+ | - | 2+ | +/- |
| no conjugate | - | - | +/- | +/- |

Selection of an anti-HSV-1-HRP final dilution in the range of 1:20 to 1:30 allows adequate differentiation of positive and negative samples.

(3) Herpes Simplex Virus Assay Sensitivity

The HSV-1 antigen was diluted over a range of 1:20 to 1:4860 in order to determine the sensitivity of the assay under conditions and procedures already disclosed in (C)(1) and (C)(2) above. Readings were taken 5 minutes after addition of color generating solution with the following results:

|                  | Color Intensity |          |
|------------------|-----------------|----------|
| HSV-1 Dilution   | Positive        | Negative |
| 1:20             | 4+              | bv       |
| 1:60             | 4+              | -        |
| 1:180            | 3+              | -        |
| 1:540            | 2+              | -        |
| 1:1620           | 1+              | -        |
| 1:4860           | +/-             | -        |
| no antigen       | bv              | -        |

CLAIMS:

1. An immunoassay for the detection of analytes in biological samples comprising the steps of:

(A) collecting or forming on an active support an immune complex having a first enzyme component comprising an analyte immunochemically bound to an antibody and a productive complex having a second enzyme component comprising at least one complement component which is bound to the immune complex;

(B) washing the active support;

(C) contacting the enzyme components on the support with signal-generating reagents whereby a substantially colorless product is formed which is soluble in the assay medium; and

(D) allowing the product to interact with the support which interaction results in the formation of a signal-emitting product associated with the support.

2. The immunoassay of Claim 1 wherein the productive complex is the product of complement fixation of an enzyme-labeled complement component selected from the group consisting of Clq, Clr and Cls, by an enzyme-labeled immune complex.

3. The immunoassay of Claim 1 wherein the analyte is selected from the group consisting of soluble organic antigens and haptens, cells, microorganisms and their products.

4. The immunoassay of Claim 1 wherein the support comprises nylon, cellulose nitrate, ethyl cellulose, modified polyamide, starch-coated materials or starch.